# EUROPÄISCHE PATENTANMELDUNG

(11) **EP 4 094 784 A1**
(43) Veröffentlichungstag der Anmeldung: **30.11.2022**
(21) Anmeldenummer: 21176137.4
(22) Anmeldetag: 27.05.2021
(51) Int. Cl.: A61M 1/00, A61M 3/02, B01D 61/00, B01D 67/00, B01D 71/56, B01D 71/72

(54) **SYSTEM ZUR ENTFERNUNG BAKTERIELLER TOXINE AUS WUNDFLÜSSIGKEIT**

(71) Anmelder: Stangl, Manfred, 82054 Sauerlach (DE); Karcz, Konrad W., 81245 München (DE)
(72) Erfinder: Stangl, Manfred, 82054 Sauerlach (DE); Karcz, Konrad W., 81245 München (DE)
(74) Vertreter: Hertin und Partner Rechts- und Patentanwälte PartG mbB

(57) **Zusammenfassung**

Die Erfindung betrifft ein Wundbehandlungssystem, welches Wundflüssigkeit über einen Abführschlauch durch eine Saugeinrichtung abpumpt und Bakterien und Endotoxine aus der abgesaugten Wundflüssigkeit über Endotoxinabsorber und/oder Filter entfernt. Über einen Zuführschlauch wird die gereinigte Wundflüssigkeit zurück in die Wunde überführt, sodass die gereinigte Wundflüssigkeit weiterhin die Wundheilung fördern kann. Dabei sind der Abführ- und Zuführschlauch fluidisch miteinander verbunden. Eine Wundeinlage wird vom erfindungsgemäßen Wundbehandlungssystem ebenso umfasst, welches insbesondere ein Silikonkissen sein kann.

## Beschreibung

Die Erfindung betrifft ein Wundbehandlungssystem, welches Wundflüssigkeit über einen Abführschlauch durch eine Saugeinrichtung abpumpt und Bakterien und Endotoxine aus der abgesaugten Wundflüssigkeit über Endotoxinabsorber und/oder Filter entfernt. Über einen Zuführschlauch wird die gereinigte Wundflüssigkeit zurück in die Wunde überführt, sodass die gereinigte Wundflüssigkeit weiterhin die Wundheilung fördern kann. Dabei sind der Abführ- und Zuführschlauch fluidisch miteinander verbunden. Eine Wundeinlage wird vom erfindungsgemäßen Wundbehandlungssystem ebenso umfasst, welches insbesondere ein Silikonkissen sein kann.

### HINTERGRUND

Offene Wunden, welche zu groß oder zu stark entzündet sind, um selbständig zu heilen, waren lange Zeit ein schwieriges Gebiet der Medizin. Auch heute stellen infizierte Wunden die Medizin vor ernsthaften Problemen. Allein in Deutschland leiden ca. 2 - 3 Millionen Menschen unter schlecht bzw. nicht heilenden Wunden (siehe [1], Abschnitt [0002]).

Grundsätzlich entsteht eine Wunde durch die Zerstörung oder Trennung von Gewebe an Haut, Schleimhäuten oder Organen. Die kann durch mechanische Einflüsse, Kälte, Hitze oder chemische Stoffe entstehen. Auch können Wunden als eine Folge einer Krankheit resultieren, z. B. als Geschwür. Durch die so entstehende Verletzung werden bestimmte Funktionen der Haut, wie z. B. die Schutzfunktion, beeinträchtigt oder gehen ganz verloren. Die Haut umfüllt den Menschen und wirkt wie ein schützender Mantel. Die Haut bewahrt innere Organe vor schädlichen Umwelteinflüssen. Ein Wunddefekt auf der Haut sollte daher schnellstmöglich und unkompliziert heilen.

Die Wundheilung allgemein, insbesondere die Wundheilung auf der Haut, gehört zu den komplexesten Vorgängen im menschlichen Körper. Die Wundheilung erfolgt in Phasen, die zeitlich überlappend nacheinander auftreten. Dabei basiert die Einteilung der Phasen auf morphologischen Leitkriterien (d. h. der Form nach) und unterscheidet sich voneinander je nach Quelle.

In [2] wird der Prozess der Wundheilung in drei Phasen unterteilt, nämlich die exsudative Phase, die proliferative Phase und schließlich die reparative Phase.

In der exsudativen Phase, die bis zu 3 Tage dauern kann, findet die Hämostase statt. Die Hämostase bezeichnet einen überlebenswichtigen Prozess, der die bei Verletzungen der Blutgefäße entstehende Blutungen zum Stillstand bringt. Ein großer Verlust von Blut aus dem Blutkreislauf wird somit verhindert und die Voraussetzung für die Wundheilung wird geschaffen. Die Hämostase lässt sich in zwei Teilvorgänge unterteilen, die jedoch miteinander in Wechselwirkung stehen. Bei der primären Hämostase, der Blutstillung, sind die Thrombozyten, welche die kleinsten Zellen des Blutes bezeichnen, die Wandzellen des betroffenen Blutgefäßes und Gewebe außerhalb des Gefäßes beteiligt. Bildlich gesprochen verengt sich zunächst das Gefäß, woraufhin sich die Thrombozyten an das "Leck" heften und untereinander verkleben und dadurch einen ersten Verschluss bilden. Bei der sekundären Hämostase, der Blutgerinnung, wird der Verschluss, der noch lose ist, über Fibrin-Fäden verstärkt. Die Wundheilung an sich wird durch Wachstumsfaktoren initiiert. Wachstumsfaktoren sind multifunktionelle Proteine, die als biochemische Mediatoren Signale zwischen Zellen vermitteln. Die Vermittlung des Signals erfolgt über eine Bindung des Wachstumsfaktors an einen spezifischen Rezeptor der Zellmembran.

In der proliferativen Phase, die ca. zwischen dem 4. und dem 7. Tag nach der Wundentstehung stattfindet, bauen sich von den Wundrändern aus Kollagenfasern entlang dem gebildeten Fibrinnetz aus und bilden neues Bindegewebe. Dies wird durch eine von Fibronektin und anderen Proteasen gebildeten Fibroblastenvermehrung bewirkt. Kollagenfasern sind Strukturproteine des Bindegewebes. Fibronektin bezeichnet ein Glykoprotein der extrazellulären Matrix, wobei die extrazelluläre Matrix der Gewebeanteil zwischen den Zellen im sogenannten Interzellularraum ist. Als Fibroblast bezeichnet man die ortsständige spezifische Zelle des Bindegewebes. Im neuen Bindegewebe lagern sich weitere Zellen ein, sodass ein körniges Granulationsgewebe entsteht, welches die Wunde von unten ausfüllt, sodass sich die Wunde immer weiter schließt.

In der reparativen Phase, die ca. ab dem 8. Tag nach der Wundentstehung beginnt, wird die Wunde durch Epithelisation verschlossen. Dabei überwachsen Epithelzellen, welche mehrlagige Zellschichten darstellen, die Wunde. Eine Voraussetzung für den komplikationslosen Ablauf der reparativen Phase ist ein ausreichend feucht-warmes Wundmilieu, das eine Einwanderung frischer Zellen begünstigt.

Die Behandlung von oberflächlichen, aber auch tiefliegenden Wunden, insbesondere von Wunden auf der Haut, hat sich in den letzten Jahren deutlich geändert. So ist ein Austrocknen von Wunden hinsichtlich der Vermeidung von Infektionen überholt, da dieses zu den Störungen der Gewebereparatur und somit zu einer verlangsamten Wundheilung führt. Es ist eher erwünscht, ein physiologisches Wachstums- und Heilungsmilieu im Wundbereich zu ermöglichen, um dadurch pathologische Wundheilungsmechanismen zu verhindern.

Ein erster Schritt in diese Richtung war die Einführung von hydroaktiven Wundauflagen zur Regulation des Feuchtigkeitsgehalts im Wundgebiet. Es sind auch schon vielfältige Produkte in diesem Bereich bekannt bis hin zur Vakuumbehandlung. Jedoch ist die Überlegenheit einzelner über andere bislang aber nicht evidenzbasiert. Verbindliche Leitlinien zur Standardisierung eines modernen Wundmanagements sind bisher auch nicht vorhanden.

Ein neuer Ansatz zur Förderung der Wundheilung wird in [3] vorgestellt. Darin wird offenbart, dass antimikrobielle Substanzen zur Endotoxinabsorption auf Wunden von Mäusen gelegt werden. Insbesondere werden Polyurethan-Schaumstoffe untersucht. Es stellte sich heraus, dass solche Schaumstoffe tatsächlich Endotoxine absorbieren und antimikrobiell wirken. Dabei bezeichnen Endotoxine einen Bestandteil der äußeren Zellmembran von gramnegativen Bakterien oder Cyanobakterien. Chemisch betrachtet, stellen sie Lipopolysaccharide dar, also thermostabile Verbindungen aus fettähnlichen- und Zuckerbestandteilen. Beim Zerfall von Bakterien werden Teile davon, eben die Endotoxine, frei und wirken toxisch. Ein großer Nachteil der in [3] vorgestellten Methode ist, dass Polyurethan Schaumstoff in der Wunde bleibt. Im schlimmsten Fall kann die Haut mit dem Schaumstoff verwachsen. Ein Entfernen des Schaumstoffes hätte dann zur Folge, dass die Wunde sich erneut öffnen würde und ein bisheriger Heilungsverlauf de facto wieder von neuem starten müsste.

In [4] wird eine Vorrichtung zur Entfernung von bakteriellen Lipopolysacchariden und Lipoteichonsäuren aus Blut oder Plasma offenbart. Dies geschieht allerdings im Zusammenhang mit einem extrakorporalen Perfusionssystem. Insbesondere ist die in [4] beschriebene Vorrichtung in einem Perfusionssystem eingebaut, nämlich wobei in einem in das Perfusionssystem integrierbaren Gehäuse ein zur selektiven Entfernung von bakteriellen Lipopolysacchariden geeignetes Hohlfasermaterial vorliegt. Es wird in [4] nirgends offenbart, dass bakterielle Toxine aus spezifischen Wundbereichen an der Haut entfernt werden.

### ZUSAMMENFASSUNG DER ERFINDUNG

Aufgabe der vorliegenden Erfindung war es, die Nachteile des Standes der Technik zu beseitigen und ein verbessertes Wundheilungssystem bereitzustellen. Insbesondere war es eine Aufgabe der Erfindung ein Wundheilungssystem anzubieten, welches auch die Endotoxine einer Wunde entfernt, ohne jedoch der Wunde weiteren Schaden hinzuzufügen.

Die erfindungsgemäße Aufgabe wird gelöst durch die Merkmale der unabhängigen Ansprüche. Vorteilhafte Ausgestaltungen der Erfindung sind in den abhängigen Ansprüchen beschrieben.

Die Erfindung betrifft daher ein Wundbehandlungssystem zur Entfernung bakterieller Toxine aus der Wundflüssigkeit umfassend einen an eine Absaugeinrichtung angeschlossenen Wundflüssigkeitsabführschlauch, dadurch gekennzeichnet,
a) dass der Abführschlauch (4) eine Matrix zur Absorption von bakteriellen Lippolysachariden (LPS) oder/und Lipoteichonsäuren (LTA) umfasst oder mit einer Matrix verbunden ist, wobei die Matrix so angeordnet ist, dass die abgesaugte Wundflüssigkeit (2) über die Matrix geleitet wird,
   und
b) dass der Abführschlauch fluidisch mit einem Wundflüssigkeitszuführschlauch verbunden ist.

Das Wundbehandlungssystem ist dazu eingerichtet, bakterielle Toxine aus der Wundflüssigkeit zu entfernen und die gereinigte Wundflüssigkeit wieder in der Wunde zurückzuführen.

Wundflüssigkeit umfasst mehrere Bestandteile, die für die Wundheilung notwendig oder vorteilhaft sind. Wundflüssigkeit umfasst dabei Wasser, Fibrine, Glukose, Lymphozyten, Makrophagen, Thrombozyten, Proteine und Wachstumsfaktoren. Durch die Zurückführung der Wundflüssigkeit werden insbesondere wundheilungsfördernde Bestandteile zurück in die Wunde gebracht, sodass die Wundheilung effektiver durchgeführt werden kann.

Wasser dient als Transportmedium und ist als fester Bestandteile zu erachten und dient mitunter dazu, der Wunde eine gewisse Feuchtigkeit zu verleihen.

Fibrine sind für die Wundheilung essenziell. Ein Fibrin ist ein hochmolekulares, nicht wasserlösliches Molekül, das bei der Blutgerinnung durch die enzymatische Wirkung von Thrombin und Fibrinogen entsteht. Fibrine bilden einen vernetzten Belag und ballen damit Blutplättchen zusammen. Durch die Wasserunlöslichkeit wird gewährleistet, dass der Fibrinbelag tatsächlich die Wunde verschließt und die Blutung stillt. Insbesondere bei chronischen Wunden ist allerdings der Fibrinbelag ein negativer Umstand, da dieser austrocknet, fest am Gewebe haften bleibt und weiterhin wasserunlöslich und dann sogar zähflüssig ist. In einem solchen Fall kann beispielsweise mit einer Wundspüllösung der Fibrinbelag gereinigt werden. Auch der Einsatz von Wundgel kann hierfür nützlich sein.

Die Glukose dient der Energieversorgung der Zellen, sodass die Wundheilung fortlaufend stattfinden kann.

Lymphozyten sind eine Untergruppe der weißen Blutkörperchen (Leukozyten), die vor allem für die Immunabwehr verantwortlich sind. Insbesondere regulieren sie die Antwort des Immunsystems auf Erreger, die durch die offene Wunde in den Blutkreislauf hineingelangen könnten. Sie bekämpfen infizierte oder entartete Körperzellen und unterstützen indirekt die Reifung von Antikörpern.

Makrophagen bewirken die Reinigung der Wunde durch Phagozytose. Anschaulich beschrieben ist Phagozytose ein aktiver Transportprozess, bei dem eine Zelle feste Partikel oder andere Zellen aus dem Extrazellulärraum aufnimmt, indem sich die Zellmembran einstülpt. Die Partikel werden dann in Vesikeln (kleine bläschenförmige Bildung im Zytoplasma) transportiert. Zudem produzieren Makrophagen Wachstumshormone, sodass neues Gewebe wachsen kann.

Proteine, wie Albumine, Fibrinogene und Globuline wirken als Transportmedium für andere Moleküle, weisen eine antienzündliche Wirkung auf und haben darüber hinaus eine blutstillende Wirkung und können ebenfalls die Immunabwehr fördern.

Eine besondere Bedeutung haben Wachtumsfaktoren bei der Wundheilung. Wachstumsfaktoren werden entweder sezerniert, d. h. von Zellen in die Wundumgebung abgegeben, oder sind membranbeständig. Sie stimulieren die Neubildung von Zellen und Gewebe. Wachstumsfaktoren lassen sich in große Familien einteilen, von denen insbesondere die FGF-Familie (Fibroblast Growth Factor), die PDGF-Familie (Platelet Derived Growth Factor), die HGF-Familie (Hepatocyte Growth Factor) einen positiven Einfluss auf die Wundheilung nehmen.

Die eben beschriebenen Bestandteile der Wundflüssigkeit sind keineswegs darauf limitiert oder beschränkt. Wundflüssigkeit ist ein komplexes Gemisch, bei dem viele Bestandteile während der Wundheilung wichtig sind.

Die Zurückführung der Wundflüssigkeit - nach dem z.B. LPS und/oder LTS entfernt wurden - stellt eine besondere und bisher unerwartet vorteilhafte Behandlungsmethode für insbesondere großflächige Wunden dar.

Erfindungsgemäß wird ein Wundflüssigkeitsabführschlauch an die Wunde gebracht, wobei der Wundflüssigkeitsabführschlauch vorzugsweise nicht direkt mit der Wunde verbunden ist, sondern eine Wundauflage auf die Wunde gelegt wird. Vorteilhafterweise wird somit die Wunde nicht direkt vom Abführschlauch berührt und weitere Folgeverletzungen werden vermieden. Über die Absaugeinrichtung wird Wundflüssigkeit aus der Wunde abgepumpt. Dies geschieht mittels der Absaugeinrichtung, welche einen Unterdruck und somit einen Sog erzeugt, sodass die Wundflüssigkeit in den Wundflüssigkeitsabführschlauch gelangt.

Im Rahmen des vorliegenden Wundbehandlungssystem wurde erkannt, dass bei der Behandlung von infizierten Wunden chromatographisch wirksame Adsorbenzien, welche bakterielle Toxine binden und negative Einflüsse dieser Toxine verhindern oder verringern können. Durch die adsorptive Bindung und damit Entfernung insbesondere der Endotoxine von gram-negativen Bakterien, der Lipopolysaccharide LPS, und auch der Toxine von gram-positiven Bakterien, der Lipoteichonsäure LTA, aus dem Wundmilieu wird es ermöglicht, im Bereich der Wunde eine erhebliche Verbesserung der pathophysiologischen Umstände herbeizuführen, sodass die natürliche Wundheilung weitestgehend ungehindert fortschreiten kann.

Bakterielle Toxine stellen eine Untermenge von mikrobiellen Toxinen dar. Im Sinne der Erfindung bezeichnet ein mikrobielles Toxin, das von Mirkoorganimsen, wie z. B. Bakterien oder Pilzen, erzeugt wird. Dabei bezeichnet ein Toxin ein Gift, welches von einem Lebewesen synthetisiert wird. Bakterielle Toxine werden in Exotoxine und in Endotoxine unterschieden.

Exotoxine sind als komplex zusammengesetzte Proteine aufgebaut. Sie sind Ausscheidungsgifte, die permanent von Bakterien ausgesetzt werden. Sie sind thermolabil, d. h. sie sind nicht gegen Hitze oder Kälte resistent.

Endotoxine bestehen aus Lipopolysacchariden und sind in der äußeren Hülle von gramnegativen Bakterien zu finden. Sie werden beim Zerfall von Mikroorganismen freigesetzt. Dadurch können sich große Mengen von Toxinen entfalten. Zudem sind Endotoxine thermostabil. Daher ist für eine verbesserte Wundheilung zielführend, dass die Endotoxine aus der Wundflüssigkeit entfernt werden, um eine schneller und allgemein bessere Wundheilung zu ermöglichen. Vorteilhafterweise wird dies mit dem erfindungsgemäßen Wundbehandlungssystem erlangt. Dabei wird Wundflüssigkeit, die bakterielle Toxine, insbesondere Endotoxine, beinhaltet, über den Wundflüssigkeitsabführschlauch über die Absaugeinrichtung angesaugt und somit aus der Wunde entfernt.

LPS und LTA sind relativ große Biopolymere, die bis zu einige Millionen Dalton Molekulargewicht aufweisen können. Die toxische Substanz im Rahmen von LPS und LTA ist aber jeweils ein Lipid mit einem Molekulargewicht von etwa 1.500 Da.

Bei LPS handelt es sich um Lipid A, welches an die Kopfseite der veresterten Zuckerpolymere von LPS gebunden ist. Bei LTA ist die eigentliche toxische Substanz ebenfalls ein Lipid, das gleiche chemische, ionische und hydrophobe Eigenschaften wie Lipid A aufweist. Lipoteichonsäuren bestehen aus einer hydrophilen Kette aus Alditolphosphaten und einem Glycolipid, welches die Kette in der Membran der Bakterien verankert.

Als Teichonsäuren werden polymere Bausteine der Zellwand grampositiver Bakterien bezeichnet. Sie könne 20 bis 40 % ihrer Zellwand-Trockenmasse ausmachen. Teichonsäuren bestehen entweder aus Ribitol-Phosphat-Polymeren, welche auch Ribitol-Teichonsäuren genannt werden oder aus Glycerol-Phosphat-Polymeren, auch unter dem Begriff Glycerol-Teichonsäuren bekannt, wobei sie kettenartig nach außen ragen. Lipoteichonsäuren sind Glycerol-Teichonsäuren, die kovalent an Glycolipiden der Zellmembran gekoppelt sind. Teichonsäuren und Lipoteichonsäuren sind starke exogene Pyrogene. Sie gehören damit zu den Stoffen, die beim Menschen nach einer bakteriellen Infektion durch grampositive Bakterien eine fieberhafte Reaktion hervorrufen. Darüber hinaus sind sie eines der Hauptfaktoren für die Entzündungsreaktion nach einer Infektion. Daher ist es erstrebenswert, dass auch LTA aus der Wunde entfernt wird.

Im Sinne der Erfindung bezeichnet Wundflüssigkeit jene Flüssigkeit, die die Wunde je nach Zustand, Größe und Sauberkeit absondert. Synonym wird auch der Begriff des Wundsekrets verwendet. Der Austritt von Wundflüssigkeit geschieht nicht immer zwingend, kommt jedoch bei den meisten Wunden vor und dient der Wundheilung. Die Wundflüssigkeit verhindert die Austrocknung, scheidet Abfallstoffe, Zellreste, Fremdmaterialien und Krankheitserreger aus und soll die Wunde mit Nährstoffen und anderen wichtigen Substanzen zur Wundheilung versorgen. Ebenfalls gibt die Wundflüssigkeit Informationen darüber, wie gut oder wie schlecht die Wundheilung verläuft. Bei einem gesunden Wundbett kommt eine klare bis honigfarbene Flüssigkeit in wässriger Form zum Vorschein. Liegt hingegen eine rote oder sogar grüne Farbe mit einem unangenehmen Geruch vor, so ist von einer Entzündung auszugehen. Bei einer kontaminierten Wunde besteht die Gefahr einer Infektion, woraus eine gestörte Wundheilung und in extremen Fällen eine Sepsis, also eine Blutvergiftung, entwickeln kann. Daher ist es von Vorteil, dass das erfindungsgemäße Wundbehandlungssystem wundheilungsstörende Faktoren, insbesondere bakterielle Toxine, aus der Wunde über das Absaugen der Wundflüssigkeit aus der Wunde entfernt.

Der Abführschlauch umfasst eine Matrix zur Absorption von bakteriellen LPS und/oder LTA, wobei die Matrix so angeordnet ist, dass die abgesaugte Wundflüssigkeit über die Matrix geleitet wird. Im Sinne der Erfindung bezeichnet eine Matrix eine Substanz, die Wundflüssigkeit, insbesondere die bakteriellen LPS und LTA umgibt und mit diesen verbunden werden kann. Dadurch ergibt ein gewisses "Anhaften" der LPS und LTA an die Matrix oder Matrixoberfläche.

Die Matrix ist beispielsweise als eine unlösliche Trägermatrix zu verstehen, die modifiziert wurde, um die Funktion der LPS/LTS-Anbindung zu ermöglichen. Beispiele für solche Trägermatrizen sind unlösliche Sepharose-Trägermatrizen, auf deren Oberfläche LPS- und/oder LTS-bindenden Substanzen immobilisiert sind. Beispiele für die Oberflächenmodifizierungen der Matrix sind ausführlicher unten benannt.

Der Abführschlauch der Wundflüssigkeit umfasst einen Schlauch, der an oder in die Wundauflage geführt ist und durch die Absaugeinrichtung Wundflüssigkeit über den Abführschlauch aus der Wunde absaugt. Das Material des Abführschlauchs kann Gummi, Silikon oder Kunststoff umfassen. Der Durchmesser des Abführschlauchs kann bis zu 2mm, 5mm, 10 mm, 20 mm, 30 mm, 30 mm, 40 mm, 50 mm, 60 mm, 70 mm oder mehr betragen. Die Länge des Zuführschlauchs kann vorzugsweise zwischen 5 cm - 140 cm betragen. Insbesondere bieten sich hierbei Schlauchsysteme an, die für Vakuumpumpe hinsichtlich des Anschlusses geeignet sind, wie beispielsweise das Schlauchset mit Bakterienfilter für die Absaugpumpe "Kataspir" der Firma Medutek. Die Längen-, Durchmesser- und Materialangaben sind hierauf nicht beschränkt und können im Handlungsbereich eines Fachmanns optimiert und/oder angepasst werden. Bevorzugt gelten diese Angaben auch für den Wundflüssigkeitszuführschlauch, wobei sie im Rahmen der Gegebenheiten angepasst werden können.

Bevorzugt ist eine Öffnung des Abführschlauchs an der Wundauflage mit einem einer Folie ö. Ä. verbunden, sodass die Wundflüssigkeit sich an der Wundauflage aufsammelt, und über den Abführschlauch durch die Absaugvorrichtung nach außen gebracht und durch den Abführschlauch geführt wird. Dadurch werden vorteilhafterweise LPS und /oder LTA aus der Wunde entfernt.

Insbesondere ist es von großem Vorteil, dass der Abführschlauch fluidisch mit einem Wundflüssigkeitszuführschlauch verbunden ist. Im Sinne der Erfindung bezeichnet der Ausdruck "fluidisch verbunden", dass ein Fluid, bevorzugt Wundflüssigkeit, vom Abführschlauch in den Zuführschlauch fließt.

Der Wundflüssigkeitszuführschlauch ist bevorzugt ein Schlauch, der von der Ausführung, Form und Struktur identisch mit Abführungsschlauch ist und Wundflüssigkeit zurück in die Wundauflage der Wunde geführt wird. Dabei wird während der Abführung der Wundflüssigkeit bakterielle Toxine, insbesondere LPS und LTA, aus der Wundflüssigkeit entfernt. Die "gereinigte" Wundflüssigkeit wird somit zurück in die Wunde bzw. der Wundauflage über den Zuführschlauch geführt. Dies ist von besonderem Vorteil, da die Wundflüssigkeit, wie eingangs beschrieben, die Wundheilung fördert.

Vorteilhafterweise wird mit dem erfindungsgemäßen Wundbehandlungssystem Wundflüssigkeit über den Abführschlauch durch die Absaugeinrichtung eingesaugt, Bakterielle Toxine aus der Wundflüssigkeit durch die darin enthaltene Matrix absorbiert und gefiltert und die Wundflüssigkeit, welche nun keine oder nur geringe Anteile von bakteriellen Toxinen aufweist, über den Rückführschlauch zurück in die Wundauflage der Wunde gebracht. Diese Kombination aus der Elimination von Endotoxin und der Rückführung von Wundsekret stellt eine wesentliche Abkehr vom Stand der Technik dar. In [3] wird lediglich die Absorptionsfähigkeit von Polyurethan-Schaumstoff beschrieben, welches auf Wunden gelegt wird. Insbesondere eine Rückführung der Wundflüssigkeit, nachdem das Endotoxin absorbiert wurde, ist darin nicht beschrieben.

Es ist auch bevorzugt, dass im Wundbehandlungssystem weitere Endotoxinabsorber und Filter, wie z. B. Bakterienfilter, angeschlossen sind, sodass eine besonders gute Entfernung von mikrobiellen Organismen aus der Wundflüssigkeit, die die Wundheilung stören, stattfinden kann. Im Sinne der Erfindung bezeichnet ein Endotoxinabsorber eine Vorrichtung, die die Matrix enthält. Die Matrix wiederrum haftet die Endotoxine und die Wundflüssigkeit fließt weiter. Filter, wie Bakterienfilter, können Bakterien filtern, welche nach der Endotoxinabsorption der Matrix vorhanden sind. Nachdem alle, fast alle oder ein großer Anteil von wundheilungsstörenden, mikrobiellen Organismen, wie z. B. Endotoxine, aus der Wundflüssigkeit entfernt werden, wird über den Rückführschlauch die gereinigte Wundflüssigkeit zurück auf die Wundauflage gebracht.

In einer bevorzugten Ausführungsform weist die Oberfläche der Matrix Cholestyramin und/oder Anionenaustauschgruppierungen z. B. aus Polykationen und hydrophobe Eigenschaften auf.

Es ist von großem Vorteil, dass sich Cholestyramin auf der Oberfläche der Matrix befindet, da es Endotoxine besonders gut bindet, sodass es einfach aus der Wundflüssigkeit entfernt wird. Vorteilhafterweise gelangen somit auch Endotoxine nicht mehr in die Wundflüssigkeit, sodass die Endotoxine am Cholestyramin gebunden bleiben.

Chemisch betrachtet ist Cholestyramin ein stark basischer Anionenaustauscher. Im Sinne der Erfindung bezeichnet ein Ionenaustauscher eine Substanz, bei denen gelöste Ionen durch andere Ionen gleichnamiger Ladung ersetzt werden können. Je nachdem welche Art von Ionen ausgetauscht werden, unterscheidet man zwischen Kationen- und Anionenaustauscher. Im Sinne der Erfindung bezeichnet eine Anionenaustauschergruppierung eine Gruppe aus zwei oder mehrerer solcher Anionenaustauscher.

Derartige Anionenaustauschergruppierungen sind besonders vorteilhaft als längere Ketten ausgestaltet mit einer Vielzahl von kationischen Gruppen, als sogenannte Tentakel. Derartige tentakelartige Fortsätze sind fähig, mehrere LPS- bzw. LTA-Moleküle zu binden, wodurch die Effizienz der Matrix und des erfindungsgemäßen Wundbehandlungssystem insgesamt nochmals gesteigert werden kann.

Für diese Modifikation mittels Tentakel werden vorzugsweise synthetische oder/und halbsynthetische oder/und natürliche Polykationenketten eingesetzt, wobei diese Ketten in linearer oder verzweigter Form vorliegen können. Derartige Kationen- bzw. Polykationenketten weisen bevorzugt tertiäre oder/und quartäre Amingruppen auf.

In einer bevorzugten Ausführungsform enthalten die Anionenaustauschergruppen Di- oder Trialkylaminoalkyl-, Di- oder Trialkylaminoaryl-, Di- oder Triarylaminoalkyl-, Di- oder Triarylaminoaryl-, Di- oder Trialkylammoniumalkyl-, Di- oder Triarylammoniumalkyl-, Di- oder Triarylammoniumaryl- und Di- oder Trialkylammoniumaryl-Reste ein oder Polymere aus positiv geladenen oder tertiäre oder quartäre Aminogruppen enthaltenden Aminosäuren, wie Polylysin, Polyarginin oder Polyhistidin oder Mischpolymere oder Derivate hiervon oder Polyethylenimin.

In einer bevorzugten Ausführungsform enthalten die Anionenaustauschergruppierungen Trimethylbenzylammonium-Reste. Eine entsprechende beispielhafte Verbindung, welche als Anionenaustauschergruppierung verwendet werden kann, ist Cholestyramin (Poly(trimethylammoniomethylstyrolchlorid-codivinylbenzol)).

In weiteren bevorzugten Ausführungsformen können weitere LPS-Adsorber zum Einsatz kommen, welche aus dem Stand der Technik bekannt sind. Im Rahmen der Kenntnisse eines Fachmanns können diese mit der erfindungsgemäßen Vorrichtung integriert werden. In [4] werden beispielsweise Hohlfasermaterialien, welche aus Polyamid, Polysulfon, Polyether, Polyethylen, Polypropylen, Polyester oder/und Mischungen solcher Polymere hergestellt werden (siehe Anspruch 4, [4]), offenbart, welche mit den eben beschrieben Anionenaustauschergruppen (siehe Ansprüche 7, 10, [4]) aufpolymerisiert werden, um die LPS zu binden.

In [5] wird offenbart, dass Polycaprolacton, ein biologisch abbaubarer Kunststoff hergestellt basierend auf Erdöl, als Nanopartikel in Pulverform LPS binden kann. Die Hinzugabe von phosphatgepufferten Salzlösungen erhöhte zudem die Bindung von LPS an die Polycaprolacton-Nanopartikel. Dieser LPS-Adsorptionsmethode kann in bevorzugten Ausführungsformen ebenfalls an die Erfindung angebracht werden, um LPS aus der Wundflüssigkeit zu entfernen und an sich zu binden.

Weitere Möglichkeiten zur LPS-Adsorption sind beispielsweise in der Tabelle 1 in [6] offenbart, welche in bevorzugten Ausführungsformen der Erfindung ebenso benutzt werden können. Diese können unter anderem durch "direkte Waschmethoden" geschehen mittels Alkaliethanol, Salpetersäure und einem bestimmten Anteil an Ethanol, die in einem Ultraschallbad behandelt werden. Mikro- und/oder Ultrafiltrationsmethoden können auch bevorzugt sein. Beide Filtrationsmöglichkeiten haben sich im Stand der Technik als zuverlässige Methode erwiesen, LPS zu filtrieren. Auch Polyglycidylmethacrylat (PGMA) kann zur LPS-Entfernung aus der Wundflüssigkeit bevorzugt sein. In weiteren bevorzugten Ausführungsformen kann N,N-Dimethylaminopropylacrylamide (DMAPAA), 2-Phasen-Mizellensysteme, Trennverfahren wie die Affinitätschromatographie, immobilisierte Metallaffinitätschromatographie, Größenausschlusschromatographie (Size-exclusion chromatography SEC), Anionenaustauschchromatographie, Kationenaustauschchromatographie und/oder immobilisiertes hydrophobe Liganden- und Umkehrphasenchromatographie (Immobilized hydrophobic ligands and reverse-phase chromatography RPC). Zur konkreten Anwendung dieser LPS-Filtrier-, Adsorptions- und/oder Bindungsmöglichkeiten wird auf [6] verwiesen. Für weitere Ausführungen zu diesen weiteren Möglichkeiten, sei auch auf [7] verwiesen.

In [8] werden basierend auf Polyethylenimin Endotoxine entfernt. Dabei wird Polyethylenimin auf mit Polyvinylalkohol beschichteten Nylonmembranen, Polyvinylalkohol und Polyethylenvinylalkohol-Hohlfaser-Membranen und Sepharose immobilisiert. In bevorzugten Ausführungsformen können auch mit diesen Methodiken LPS aus der Wundflüssigkeit entfernt werden. Für weitere Details wird auf [8] verwiesen.

In einer Ausführungsform der Erfindung ist das System so konfiguriert, dass der Abführschlauch (4) eine Matrix zur Absorption von bakteriellen Lippolysachariden (LPS) oder/und Lipoteichonsäuren (LTA) umfasst, oder mit einer Matrix verbunden ist, wobei die Matrix so angeordnet ist, dass die abgesaugte Wundflüssigkeit (2) über die Matrix geleitet wird.

Beispielsweise kann die Matrix als Teil des Schlauches sein. Alternativ kann die Matrix in einer Kassette oder in einem Adsorber angeordnet sein, welches dann mit dem Abführschlauch funktionell verbunden ist. Im Wesentlichen muss die Wundflüssigkeit, die im Abführschlauch von der Wunde weggeführt wird, über die Matrix geleitet werden, um die Toxine zu entfernen. Die Matrix kann daher, in einer Ausführungsform, als ersatzbarer Adsorber oder Kassette vorhanden sein, wobei es beispielsweise auch eine Halterung für die Kassette im oder am Abführschlauch vorhanden ist, so dass die Kassette in einfacher Weise ausgetauscht werden kann, ohne die Schläuche, Pumpe oder sonstige Komponenten des Systems neu anbinden oder koppeln zu müssen. Der Fachmann ist in der Lage solche funktionell verbundenen bzw. fluidisch verbundenen Teile mit einander zu koppeln, um Systeme mit ersatzbaren Teilen zu ermöglichen.

In einer bevorzugten Ausführungsform ist die Absaugeinrichtung eine Vakuumpumpe. Die Vakuumpumpe dient dazu, Wundsekret aus der Wunde abzusaugen und es somit in den Abführschlauch zu zuführen. Dabei wird durch die Vakuumpumpe ein Unterdruck im Wundbereich durch einen Sog aufrechterhalten.

Das Funktionsprinzip ist auch in anderen Gebieten der Wundheilung bekannt. Die Vakuumpumpe erzeugt einen Unterdruck in der Wunde. Dies kann bevorzugt auch ein örtlich begrenzter Unterdruck sein. Dies ist dahingehend von Vorteil, dass nicht eine zu große Menge an Wundflüssigkeit abgesaugt wird, sodass ein Stau an Wundflüssigkeit innerhalb des Abführschlauchs vermieden wird. Es kann auch bevorzugt sein, dass die gesamte Wundfläche einem Unterdruck durch die Vakuumpumpe ausgesetzt ist. Dies weist den Vorteil auf, dass besonders schnell eine Wundheilung erfolgen kann.

Im Sinne der Erfindung bezeichnet ein Unterdruck einen relativen Druck, der in Bezug auf den Druck der Umgebung angegeben wird. Im Falle eines Unterdrucks ist der Druck eines Systems niedriger als der Druck, der in der Umgebung vorliegt. Durch das Vorliegen eines Unterdrucks, bevorzugt verursacht die Vakuumpumpe als Absaugeinrichtung, wird ein Sog verursacht. Der Sog ist die Bezeichnung für die Saugwirkung, sodass durch die Vakuumpumpe Wundflüssigkeit in den Abführschlauch geführt werden kann.

Es kann auch bevorzugt sein, dass nicht nur eine, sondern zwei, drei, vier oder mehr Vakuumpumpen eingesetzt werden. Dies ist dahingehend vorteilhaft, dass ein besonders effektives Vakuum erzeugt werden kann. Mehrere Vakuumpumpen können auch der Größe des Abführschlauchs angepasst sein. Je größer der Abführschlauch angepasst ist, desto "mehr" Vakuum muss erzeugt werden, um ein Unterdruck zu erzeugen, sodass ein Sog der Wundflüssigkeit erreicht wird.

Bevorzugt weist die Vakuumpumpe Anschlüsse auf, sodass der Abführ- und Zuführschlauch mit der Vakuumpumpe verbunden ist. Dies kann bevorzugt eine fluidische Verbindung sein. Ebenso ist es bevorzugt, dass die Vakuumpumpe weitere Bauteile umfasst, die den Druck, also den Unterdruck, regulieren. Dies ist von Vorteil, um den Strom der Wundflüssigkeit zu drosseln. Ein zu schneller Strom der Wundflüssigkeit könnte zu einem Stau innerhalb der Schläuche führen, ein zu langsamer Strom könnte es nicht weit genug in den Abführschlauch schaffen, sodass die Wundflüssigkeit somit auch nicht in den Zuführschlauch und somit zurück in die Wunde könnte. Daher sind weitere Komponenten zur Regulierung des Unterdruckes vorteilhaft.

Es kann bevorzugt sein, dass die Vakuumpumpe elektrisch und/oder mechanisch betrieben wird. Eine elektrisch betriebene Vakuumpumpe hat den Vorteil, dass eine besonders einfache Handhabung der Vakuumpumpe möglich ist. Eine mechanisch betriebene Vakuumpumpe kann vorteilhafterweise ohne elektrischen Strom in Betrieb genommen werden. Dies hat den großen Vorteil, dass die Vakuumpumpe auf elektrischen Strom nicht angewiesen ist und außerhalb der Nähe von Steckdosen eingesetzt werden kann.

Es ist bekannt, dass der Begriff des "Vakuums" im Kontext der Wundheilung nicht als einen vollständig fluidleeren Raum anzusehen ist, sondern als einen räumlichen Bereich, bevorzugt innerhalb des Schlauchsystems, welches den Abführ- und Zuführschlauch des erfindungsgemäßen Wundheilungssystems umfasst, der bevorzugt einem Grobvakuum oder bevorzugt einem Feinvakuum entspricht. Im Sinne der Erfindung bezeichnet ein Grobvakuum einen Druckbereich, der niedriger als der umgebende Luftdruck ist und bis zu 1 mbar (Millibar) betragen kann. Ein Feinvakuum bezeichnet im Sinne der Erfindung eine Druckbereich von 1 mbar bis hin zu 10⁻³ bar.

Es ist bekannt, dass standardmäßige Umgebungsluftdruck einen Druck von 1013,26 mBar bezeichnet. Im Stand der Technik ist auch bekannt, die Druckeinheit mbar in hPa zu nennen, da eine Umrechnung nichts ändert und der Zahlenwert gleichbleibt. Aber auch andere Druckeinheiten können für die Beschreibung des Vakuums angewendet werden. Insbesondere in der Medizin ist es bekannt, den Druck in der Einheit Millimeter Quecksilbersäule (mmHg) anzugeben.

Beispielsweise kann als Vakuumpumpe die Absaugpumpe "Kataspir" von Medutek dienen. Diese Pumpe ist ein kompaktes Absauggerät von Sekreten für den stationären Gebrauch und kann vorteilhafterweise sowohl bei Erwachsenen als auch bei Kindern zum Einsatz kommen. Alle Komponenten befinden sich in einem schlagsicheren Kunststoffgehäuse. Die Absaugeinrichtung ist zudem geräuscharm und ist somit für den Dauerbetrieb einsetzbar.

In einer bevorzugten Ausführungsform umfasst der Abführschlauch einen Filter, der so angeordnet ist, dass abgesaugte Wundflüssigkeit vom Abführschlauch durch den Filter geleitet wird, und der Filter bevorzugt zum Filtern von Bakterien geeignet ist.

Es hat sich herausgestellt, dass der Einsatz von Filter für das erfindungsgemäße Wundbehandlungssystem sehr vorteilhaft ist. Durch Filter können neben Mikroorganismen wie Bakterien auch Partikel gefiltert werden, die während der Entstehung der Wunde zustande gekommen sind. Es ist nicht unüblich, dass beispielsweise bei einer Schnittwunde kleinere Hautreste oder sogar Segmente aus der Kleidung in die Wunde hineingelangen. Durch den Einbau eines Filters können die abgeführte Wundflüssigkeit diese kleineren Objekte mitnehmen und sich am Filter ablagern. Das ist von großem Vorteil, da aus der Wunde auch "größere" Partikel entfernt werden. Insbesondere wird durch den Einsatz des Filters ermöglicht, dass die wieder zurückgeführte Wundflüssigkeit über den Zuführschlauch gereinigt ist von diesen Partikeln.

Bevorzugt ist der Filter dazu geeignet Bakterien zu filtern. Der Filter kann also derart ausgebildet sein, dass neben größeren Partikeln auch Bakterien gefiltert werden. Dies ist von besonderem Vorteil, da Bakterien die Wundheilung stark behindern können. Insbesondere können Bakterien die Wunde infizieren. Besonders gefährlich wird es, wenn bestimmte Bakterien durch offene Wunden in den Körper gelangen, z. B. Staphylokokken. Zwar sind harmlose Fälle bekannt, wo durch Staphylokokken z. B. leichte Haarbalg-Entzündungen entstanden sind, aber auch lebensbedrohliche Fälle wie Blutvergiftungen. Vor allem Staphylokokken sind sehr anpassungsfähig und antibiotikaresistent. Das kann auch andere Arten von Bakterien betreffen. Durch den Filter werden Bakterien von der Wundflüssigkeit herausgefiltert, sodass durch den Zuführschlauch von Bakterien gereinigte Wundflüssigkeit zurück in die Wunde fließen kann.

Gerade die bevorzugte Kombination aus einem Endotoxinabsorber und einem Filter, insbesondere einem Bakterienfilter, sorgt für eine besonders gute Reinigung der Wundflüssigkeit, welche dann zurück in die Wunde fließen kann. Da die Wundflüssigkeit einen entscheidenden Einfluss auf die Wundheilung nimmt, ist es von hohem Interesse, dass die Wundflüssigkeit von Endotoxinen, aber auch Bakterien und anderen störenden Mikroben und/oder Partikeln gereinigt wird. Das Risiko einer Wundinfektion wird durch den Einbau von Filtern, bevorzugt Bakterienfilter, extrem verringert, was von sehr großem Vorteil ist.

Bevorzugte Filter weisen eine Porenweite von 0,01 µm bis 10 µm auf. Bevorzugte Filter für Bakterien haben eine bevorzugte Porenweite zwischen 0,01 µm und 1 µm, beispielsweise 0,1, 0,2, 0,3, 0,4 oder 0,5 µm. Bekannte und kommerziell erhältlichen Filter haben eine Porenweite von 0,22 bis 0,45 µm; und beispielsweise weisen kommerziell erhältlichen Filters eine Dicke von z.B. zwischen 10 und 1000 µm, vorzugsweise zwischen 100 und 500, z.B. 150 µm auf.

Beispielsweise kann ein Bakterienfilter vom Typ Filterkerze für Bakterien (0,01 - 1,2 µm) der Firma Techno Filter verwendet werden. Alternativ könnte ein Hydrophober Bakterienfilter, z.B. für ARDO Chirurgiesäuger, eingesetzt werden. Auch der Iso-Gard Bakterien-/Virenfilter, der ein kompakter, steriler, hydrophober Filter ist, könnte eingesetzt werden. Der Einsatz von verschiedenen Bakterienfiltern liegt im Ermessungsspielraum des Fachmanns und kann je nach Anwendungsfall angepasst und/oder optimiert werden.

Es kann auch bevorzugt sein, dass die Filter, insbesondere die Bakterienfilter, neben der Filtrationswirkung auch und/oder nur eine Absorptionswirkung umfasst. Hierbei bezeichnet die Absorptionswirkung des Filters im Sinne der Erfindung die Fähigkeit des Filters, auf ihre Oberfläche oder in ihr Volumen Bakterien und/oder Partikel mit aufzunehmen und an sich zu binden. Dadurch ist es für die Bakterien und/oder Filter unmöglich, den Filter zu verlassen.

Vorzugsweise umfassen die Bakterienfilter gesintertes Glas, unglasiertes Porzellan, Kieselgur, und Cellulosederivaten umfassen. Im Stand der Technik ist die Verwendung von gesintertem Glas auch als Fritte, unglasiertes Porzellan für Chamberland-Filter, Kieselgur für Berkefeld-Filter und Cellulosederivaten für Membranfilter bekannt. Diese sind besonders einfach in Ihrer Bauweise und Konstruktion, sodass sie auch einfach und kostengünstig an das Abführschlauch des erfindungsgemäßen Wundbehandlungssystem integriert werden können.

Es kann auch bevorzugt sein, dass mehrere Filter angebracht sind, um eine für eine besonders gute Filtration von bevorzugt Bakterien zu sorgen. Die Zahl angebrachter Filter, insbesondere für Filter für Bakterien, ist nicht auf eine feste Zahl beschränkt und kann je nach Situation von dem Anwender angepasst werden.

In einer bevorzugten Ausführungsform ist ein Wundflüssigkeitsreservoir fluidisch mit dem Abführ- und Zuführschlauch verbunden.

Im Sinne der Erfindung bezeichnet ein Wundflüssigkeitsreservoir eine Vorrichtung, welche derart ausgestaltet ist, dass sie ein gewisses Volumen an Wundflüssigkeit aufsammeln kann. Bevorzugt ist das Wundflüssigkeitsreservoir fluidisch mit dem Abführ- und Zuführschlauch verbunden. Vorteilhafterweise kann ein Anteil der Wundflüssigkeit vom Abführschlauch in das Wundflüssigkeitsreservoir hineingelangen und der andere Anteil über den Zuführschlauch zurück zur Wunde gebracht werden, um weiterhin die Wundheilung zu fördern.

Es ist von Vorteil, dass bevorzugt ein Wundflüssigkeitsreservoir im erfindungsgemäßen Wundbehandlungssystem eingebaut sein kann, da die Menge an zurückgeführter Wundflüssigkeit verringert werden kann. Eine zu hohe Menge an zurückgeführter Wundflüssigkeit könnte die Wunde weiter ausfeuchten, was für die Wundheilung störend wäre. Dagegen hilft vorzugsweise ein Wundflüssigkeitsreservoir dabei einen Anteil der Wundflüssigkeit vorerst zu verstauen.

In einer bevorzugten Ausführungsform ist das Wundflüssigkeitsreservoir, der Abführ- und der Zuführschlauch über einen Dreiwegeanschluss verbunden, wobei der Dreiwegeanschluss bevorzugt ein Ventil zur Leitung der Wundflüssigkeit umfasst.

Im Sinne der Erfindung bezeichnet ein Dreiwegeanschluss bevorzugt eine fluidische Verbindung, die einen Eingang umfasst, welche bevorzugt vom Abführschlauch gebildet wird, und eine bevorzugt zwei Ausgänge. Eines der Ausgänge verläuft vorzugsweise in das Wundflüssigkeitsreservoir, der andere Ausgang verläuft entsprechend bevorzugt über den Rückführschlauch bevorzugt zurück zum Wundbereich.

Es kann auch bevorzugt sein, dass der Dreiwegeanschluss ein Ventil umfasst. Vorteilhafterweise ist es mit dem Ventil möglich, die Menge an Wundflüssigkeit zu regulieren, welche zurück zur Wunde fließen soll. In bevorzugten Varianten kann das Verhältnis von zurückgeflossene Wundflüssigkeit und Wundflüssigkeit in das Wundflüssigkeitsreservoir von 1 zu 100 bis 10 zu 1 sein, vorzugsweise von circa. 1 zu 1 bis 1 zu 10. Je nach Größe und Tiefe der Wunde kann die Rückführung der Wundflüssigkeit zur Wunde angepasst werden. Der Fachmann kann abschätzen, wie gefährlich die Wunde für die betroffene Person ist und daher auch abwägen, wieviel Anteil an Wundflüssigkeit zurück zur Wunde fließen sollte. Bevorzugt kann dies über das Ventil reguliert werden, was somit von großem Vorteil ist.

Es kann auch bevorzugt sein, dass der Dreiwegeanschluss selbst als ein Ventil ausgestaltet ist. Dies ist mit dem Vorteil verknüpft, dass der Dreiwegeanschluss sich als besonders einfach und kompakt in der Ausgestaltung zeigt und auch besonders einfach im erfindungsgemäßen Wundbehandlungssystem integriert werden kann.

In einer bevorzugten Ausführungsform des erfindungsgemäßen Wundbehandlungssystem wird eine Wundabdeckungsvorrichtung umfasst, die geeignet ist zur Aufnahme der Ab- und Zuführschläuche, wie beispielsweise eine Wundabdeckungsfolie.

Im Sinne der Erfindung bezeichnet eine Wundabdeckungsvorrichtung eine Wundauflage, die die Wunde vor Keimen, Schmutz und/oder äußeren Einflüssen schützt. Die Begriffe Wundabdeckungsvorrichtung und Wundauflage können synonym verwendet werden. Im Stand der Technik sind sogenannte inaktive Wundauflagen wie z. B. Mullkompressen oder Vliesstoffe bekannt. Sie saugen Blut und Wundflüssigkeit auf und bieten auch einen gewissen Schutz vor Verschmutzung und anderen Reizen. Allerdings haben Stoffe wie Mull und Vlies den großen Nachteil, dass sie bei feuchten, insbesondere großflächigen Wunden zu Verklebungen neigen. Bei großflächigen Wunden ist der Verbandswechsel nicht nur oftmals schmerzhaft für den Patienten, sondern häufig leidet auch das darunter neu gebildete Hautgewebe.

Wie eingangs erläutert, erleichtert ein feuchter Wundgrund den Heilungsprozess, weshalb man mittlerweile darum bemüht ist, die Wunde nicht austrocknen zu lassen. Daher sind sogenannte interaktive Wundauflagen dazu geeignet, die feuchte Wundheilung zu unterstützen. Interaktive Wundauflagen umfassen Wundabdeckungsfolien, Alginate, Hydrogele, Hdyrokolloide, Polyurethanschaumstoffe, Laminate und/oder Hydrofasern.

Wundabdeckungsfolien sind vorzugsweise dampf- und luftdurchlässig und zudem durchsichtig. Letzteres hat den Vorteil, dass der Anwender des erfindungsgemäßen Wundbehandlungssystems die Wunde gut beobachten und mögliche Infektionen frühzeitig erkennen kann, ohne die Wundabdeckungsfolie entfernen zu müssen, was von großem Vorteil ist. Insbesondere ist eine Wundabdeckungsfolie aufgrund des luftdichten Verschlusses von besonderem Vorteil.

Alginate werden aus Braunalgen gewonnen und sind reich an Kalzium- und Natriumionen. Sie binden das Wundsekret zu einem Gel und schaffen dadurch ein feuchtes Milieu. Die Alginat - Wundversorgung bietet sich bei stark nässenden, infizierten Wunden an. Für trockene Wunden hingegen ist sie nicht geeignet, da es leicht zum Verkleben kommt. Entfernt man dann den mit dem Wundgrund verklebten Alginatverband, werden neugebildete Epithelzellen abgerissen und die Heilungsdauer verzögert sich.

Hydrogele führen trockenen Wunden wieder Feuchtigkeit zu und weichen verschorfte Beläge auf. Das ist zum Beispiel bei trockenen Nekrosen nützlich. Nicht verwendet werden darf die Hydrogel-Wundversorgung bei infizierten Wunden.

Auch Hydrokolloide schaffen ein feuchtes Wundmilieu mit konstanter Temperatur. Wundauflagen mit Hydrokolloiden bestehen aus quellfähigen Partikeln (Gelatine, Cellulose etc.) und einer Abdeckfolie. Diese ist durchlässig für Gase, nicht aber für Wasser, Schmutz und Keime. Kommt die Hydrokolloidschicht mit Wundsekret in Kontakt, verflüssigt sie sich und bildet ein gelbliches, zähes Gel.

Schaumstoffverbände bestehen aus zwei Anteilen: einer Polyurethanfolie, die wasserabweisend ist, aber Wundsekret nach außen abfließen lässt, sowie dem eigentlichen Polyurethanschaum. Dieser kann große Mengen Wundsekret aufsaugen. Daher eignet sich diese Art der Wundversorgung besonders gut für stark nässende Wunden.

Ein weiterer geeigneter Vertreter für nässende Wunden ist das Polyacrylatkissen (Laminat). Diese Wundauflagen haben aber nicht nur Saugwirkung. Sie werden außerdem nach dem Aufbringen mit Kochsalzlösung getränkt, dass sie in den nächsten Stunden kontinuierlich in die Wunde abgeben. Durch diese automatisch ablaufende Spülung wird die Wunde gereinigt. So werden zum Beispiel trockene, abgestorbene Gewebeschichten (Nekrosen) aufgeweicht und schonend abgelöst.

Beispielsweise kann als Wundabdeckungsfolie der Fixierpflaster Suprasorb F der Firma Lohmann und Rauscher genutzt werden, welcher wasserdicht, elastisch, transparent und selbstklebend ist. Ein weiteres Beispiel ist die transparente Wundfolie Höga-Derm-Roll des Unternehmens höga, welcher durchsichtig und selbstklebend ist. Solche Folien, oder Folien aus den gleichen, ähnlichen oder funktionsanalogen Materialen, können selbstverständlich angepasst werden, sodass sie zur Aufnahme der Ab- und Zuführschläuche geeignet sind. Die Wahl weiterer Wundabdeckungsfolien liegt im Kenntnisbereich des Fachmanns und kann von ihm angepasst werden.

In einer bevorzugten Ausführungsform umfasst das erfindungsgemäße Wundbehandlungssystem eine Wundeinlage, wie beispielsweise einen Schwamm oder ein Silikonkissen.

Im Sinne der Erfindung bezeichnet eine Wundeinlage eine Einlage, die auf die Wunde gelegt wird. Ist die Wunde tief, kann auch davon gesprochen werden, dass die Wundeinlage in Wunde getan wird. Auch die Wundeinlage verhindert das Eindringen von Fremdkörpern in die Wunde. Des Weiteren wird auch Blut und ein gewisser Anteil der Wundflüssigkeit von der Wundeinlage aufgenommen.

Durch die Wundauflage wird die Wundeinlage auf die umgebende Haut aufgeklebt. Das Aufkleben sollte bevorzugt luftdicht stattfinden. Im Sinne der Erfindung bezeichnet eine luftdichte Verklebung eine Verklebung, wonach ein Austausch mit Luft der Umgebung nicht stattfindet. Vorzugsweise ist die Wundabdeckung gespannt beim Aufkleben mit der umgebenden Haut, sodass die Wundeinlage keine Bewegungen innerhalb der Wunde durchführt. Dies würde sonst zum Nachteil für die Wundheilung sein.

Eine bevorzugte Wundeinlage ist ein Schwamm. Ein Schwamm als Wundeinlage ist bevorzugt, da es Wundflüssigkeit und Blut aufsammeln kann. Insbesondere ist ein Schwamm fähig, Wundflüssigkeit und Blut zu speichern. Mit dem Schwamm wird ein Ende des Abführschlauchs verbunden und anschließend werden vorzugsweise der Abführschlauch und der Schwamm mit der Wundauflage auf die die Wunde umgebende Haut derart aufgeklebt, dass es luftdicht verschlossen ist. Durch die Fähigkeit des Schwammes, Wundflüssigkeit aufzusammeln und zu speichern, wird Wundflüssigkeit aus der Wunde in den Schwamm geführt. Da aber der Schwamm eine begrenzte Speicherkapazität aufweist, wird die übersteigende Menge an Wundflüssigkeit über den Abführschlauch abgesaugt. Der für die Absaugung verantwortliche Unterdruck wird durch die Absaugeinrichtung, bevorzugt eine Vakuumpumpe, erzeugt.

Zwar wird ein Schwamm von einer Wundeinlage des erfindungsgemäßen Wundbehandlungssystems umfasst, allerdings geht mit einem Schwamm ebenfalls ein Nachteil einher. Während der Wundheilung könnte es passieren, dass die Haut mit dem Schwamm verwächst. Beim Entfernen des Schwammes würde die Wunde weitere Verletzungen zu sich ziehen und die Wundheilung wäre wieder gestört und verlangsamt.

Daher ist ein Silikonkissen als Wundeinlage besonders bevorzugt. Vorteilhafterweise haftet ein Silikonkissen nicht an der Haut. Somit kann die Haut nicht mit dem Silikonkissen verwachsen. Es war überraschend, dass der Silikonkissen für die Haut sehr gut verträglich ist und der Patient hiervon keine zusätzlichen Schmerzen verspürt. Es ist auch bevorzugt, dass das Silikonkissen transparent oder transparente Bereiche aufweist. Damit wird eine Sicht zur Wunde vorteilhaft ermöglicht. Es ist ebenfalls von Vorteil, dass der Patient durch das Silikonkissen als Wundeinlage einen höheren Komfort verspürt. Das liegt daran, dass der Silikonkissen eine sehr leichte, aber trotzdem strapazierbare Ausgestaltung aufweist. Die besonders leichte Ausgestaltung kommt durch die geringe Masse des Silikonkissens zustande. Ein übermäßiger Austritt an Wundflüssigkeit aus der Wunde führt keineswegs zu einer Zerstörung des Silikonkissens.

Beispielsweise kann das Coloplast Silicone Schaumverband Multishape als Silikonkissen verwendet werdet (Herstellungsfirma Coloplast). Dieser weist eine Fläche von 14 x 19,5 cm² auf und ist als weicher, flexibler Schaumverband mit hohem Absorptionsvermögen und sanfter Silikonhaftung bekannt. Ein weiteres Beispiel ist die Biatain Contact Silikon-Wundkontaktauflage, die ebenfalls von der Firma Coloplast hergestellt wird und 5 x 7,5 cm² groß ist. Die spezifische Wahl der Wundeinlage, insbesondere des Silikonkissens und der Maße, ist vom Fachmann je nach Größe und Grad der Wunde anpassbar.

Des Weiteren kann es bevorzugt sein, dass das erfindungsgemäße Wundbehandlungssystem in Kombination mit einer Spülflüssigkeit zum Einsatz kommt. Vorzugsweise kann die Spülflüssigkeit in die Wunde injiziert werden und/oder sich innerhalb des Abführschlauches und/oder innerhalb des Zuführschlauches befinden. Mithilfe der Spülflüssigkeit kann vorteilhafter die Wunde von Wundbelag, beispielsweise durch Fibrine, Keimen, einen Biofilm, Exsudat und/oder Zelltrümmern gebildet, gereinigt.

Im Sinne der Erfindung bezeichnen Spülflüssigkeiten Flüssigkeiten, die im Rahmen von diagnostischen oder therapeutischen Maßnahmen zur Spülung verwendet werden. Eine Spülung bezeichnet im Sinne der Erfindung Verfahren, bei denen Wunden, innere Organe, Körperhöhlen und/oder Gewebebezirke mit Spülflüssigkeiten benetzt und/oder durchströmt werden, so dass dort befindliche Substanzen, Sekrete oder Fremdkörper gelöst und/oder weggeschwemmt werden. Abhängig von ihrem Einsatzgebiet können Spülflüssigkeiten sehr unterschiedlich zusammengesetzt sein und neben Wasser und Elektrolyten u.a. Antiseptika oder Arzneistoffe enthalten. Werden sie in den Körper eingebracht, müssen sie in der Regel steril und biologisch verträglich sein. Einfache Spülflüssigkeiten können beispielsweise Wasser, isotonische Kochsalzlösungen und/oder Ringer-Lösungen sein. Spülflüssigkeiten mit antiseptischer oder denaturierender Wirkung können beispielsweise eine Wasserstoffperoxid-Lösung, Alkohol und/oder eine Chlorhexidin-Lösung sein.

In einer weiteren bevorzugten Ausführungsform ist das Wundbehandlungssystem dadurch gekennzeichnet, dass der Abführschlauch (4) und der Zuführschlauch (5) so konfiguriert sind, dass die an Wunden innerhalb eines Körpers angebracht werden können, um Wundflüssigkeit (2) aus einer inneren Wunde durch den Abführschlauch (4) über die Matrix zu leiten, und anschließend durch den Zuführschlauch (5) an die innere Wunde zu befördern. Beispielsweise kann die Vorrichtung daher komplett oder teilweise steril sein, aus Bestandteilen hergestellt werden, die im Inneren des Körpers verträglich sind, und/oder mit einem Klebstoff versehen werden, der im Inneren eines Körpers anzuwenden wäre.

Topografisch können Wunden in innere und äußere Wunden unterschieden werden. Äußere Wunden sind vorzugsweise auf der Hautoberfläche anzutreffen, wobei sie auch in die Hautoberfläche hindurch reichen können, während innere Wunden sich bevorzugt innerhalb des menschlichen Körpers befinden und vorzugsweise bei inneren Organen vorzufinden sind. Insbesondere können Blutungen nach innen in den Körper bei inneren Wunden einbluten. Innere Wunden sind beispielsweise bei Schuss- oder tiefen Stichwunden durch Waffen oder auch bei spießenden Knochenbrüchen und bei Rasanztraumen, beispielsweise bei Autounfällen, zu beobachten. Gefürchtet sind hierbei einerseits ausgeprägte Blutungen durch Gefäß- oder Herzverletzungen oder aufgrund von Einrissen in Milz oder Leber. Zudem drohen Infektionen durch Eröffnung von keimbesiedelten Hohlorganen wie Darm oder Harnblase. Lebensbedrohende Funktionsstörungen bei Verletzungen von Gehirn, Herz oder Lunge können ebenfalls bei solchen Verletzungen anzutreffen sein. Insbesondere können also innere Wunden sich innerhalb einer Körperhöhle befinden. Eine Körperhöhle bezeichnet einen Hohlraum innerhalb eines Körpers, wobei dieser Begriff auch irreführend sein kann, da Körperhöhlen auch von inneren Organen ausgefüllt sein können.

Daher ist es von besonderem Vorteil, dass das erfindungsgemäße Wundbehandlungssystem nicht auf äußere Wunden beschränkt ist, sondern auch bei der Behandlung innerer Wunden eingesetzt werden kann. Insbesondere bei inneren Wunden ist eine Förderung der Wundheilung durch die erschwerte Zugänglichkeit schwierig. Das erfindungsgemäße Wundbehandlungssystem fördert die Wundheilung bei inneren Wunden, indem Wundflüssigkeit aus der inneren Wunde über Abführschlauch abgeführt wird, insbesondere bakterielle Toxine durch die Matrix aus der Wundflüssigkeit entfernt werden und gereinigte Wundflüssigkeit zurück in die innere Wunde durch den Zuführschlauch befördert wird.

Zudem kann es auch bevorzugt sein, dass das erfindungsgemäße Wundbehandlungssystem bei äußeren und insbesondere auch bei inneren Wunden während einer Operation eingesetzt wird. Innere Wunden können nämlich beispielsweise auch bei der Entfernung von Zysten, Tumoren etc. auftreten. Damit kann durch den Einsatz des erfindungsgemäßen Wundbehandlungssystems bevorzugt bereits während einer Operation die Wundheilung gefördert und der gesamte Heilungsprozess vorteilhafterweise beschleunigt werden.

Ein weiterer Aspekt der Erfindung betrifft einen Kit zur Entfernung bakterieller Toxine aus einer Wundflüssigkeit mit einem Wundbehandlungssystem nach einem oder mehreren der vorhergehenden Ansprüche, umfassend:
a. ein Wundflüssigkeitsabführschlauch, wobei der Abführschlauch eine Matrix zur Absorption von bakteriellen Lippolysachariden (LPS) oder/und Lipoteichonsäuren (LTA) umfasst, oder mit einer Matrix verbunden ist, wobei die Matrix so angeordnet ist, dass nach Zusammenbau des Wundbehandlungssystems die abgesaugte Wundflüssigkeit über die Matrix geleitet wird,
b. ein Wundflüssigkeitszuführschlauch,
c. eine Wundabdeckungsvorrichtung, die geeignet ist zur Aufnahme der Ab- und Zuführschläuche, wie beispielsweise eine Wundabdeckungsfolie, und
d. optional ein Bakterienfilter, und
e. optional eine Wundeinlage, wie beispielsweise einen Schwamm oder ein Silikonkissen

Der erfindungsgemäße Kit enthält im Wesentlichen alle Komponenten, die für die Durchführung des erfindungsgemäßen Verfahrens notwendig sind. Die Bakterienfilter und/oder die Wundeinlage können optional mit dem oder im Kit bereitgestellt werden, sodass es für medizinische Personal keinen Mehraufwand verursacht, alle notwendige Teile des Kits zusammenzustellen und somit das erfindungsgemäße Verfahren durchzuführen. Beispielsweise ist eine entsprechende Pumpe bereits vorhanden, diese kann z.B. im Krankenhaus oder zu Hause bei einem Patienten bereits vorhanden sein. Im Kit sind vorzugsweise die Verbrauchsteile enthalten, z.B. saubere und vorzugsweise sterilen Komponenten, umfassend den Wundflüssigkeitsabführschlauch mit der Matrix zur Absorption von bakteriellen Lippolysachariden (LPS) oder/und Lipoteichonsäuren (LTA), den Wundflüssigkeitszuführschlauch sowie eine Wundabdeckungsvorrichtung, die geeignet ist zur Aufnahme der Ab- und Zuführschläuche.

Ein weiterer Aspekt der Erfindung betrifft ein Verfahren zur Entfernung bakterieller Toxine aus einer Wundflüssigkeit, umfassend die Verwendung eines erfindungsgemäßen Wundbehandlungssystems und/oder eines erfindungsgemäßen Kits. Vorzugsweise wird das erfindungsgemäße Verfahren nicht direkt am menschlichen Körper durchgeführt. In einer Ausführungsform der Erfindung wird das Verfahren an einem bereits aufgebauten System durchgeführt. In einer Ausführungsform der Erfindung betrifft das Verfahren die Entfernung bakterieller Toxine aus der Wundflüssigkeit, die bereits aus der Wunde isoliert wurde. In einer Ausführungsform der Erfindung betrifft das Verfahren bevor eine (weitere) Interaktion mit dem menschlichen Körper stattfindet. Die Entfernung bakterieller Toxine aus der bereits abgesaugten Wundflüssigkeit, durch die Absorption von bakteriellen Lippolysachariden (LPS) oder/und Lipoteichonsäuren (LTA) an der Matrix, und die Zurückführung zum Wundflüssigkeitszuführschlauch, ohne direkt mit dem Körper zu interagieren, stellt eine Ausführungsform der Erfindung dar.

Sämtliche Merkmale des erfindungsgemäßen Wundbehandlungssystems sind ebenfalls so zu verstehen, als sie zusätzlich im Zusammenhang mit dem erfindungsgemäßen Kit und/oder mit dem erfindungsgemäßen Verfahren offenbart sind. Die Merkmale des Systems können daher für eine Definition des Kits bzw. des Verfahrens herangezogen werden.

Das erfindungsgemäße Wundbehandlungssystem soll im Folgenden anhand von Beispielen und Figuren erläutert werden, ohne auf diese Beispiele oder Figuren beschränkt zu sein.

### FIGUREN

### Kurzbeschreibung der Abbildung

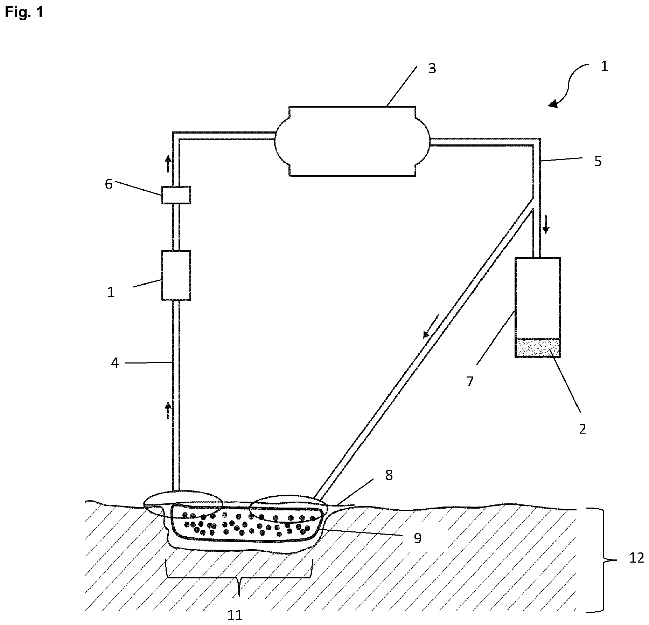

- **Fig. 1**: Darstellung eines bevorzugten Wundbehandlungssystems

### Detaillierte Beschreibung der Abbildung

**Fig. 1** ist eine Darstellung eines bevorzugten Wundbehandlungssystem **1.** Sie kommt dann zum Einsatz, wenn eine Wunde entsteht. Eine Wunde ist eine Verletzung einer Haut **11,** mit oder ohne Gewebeverlust. Wie in den obigen Ausführungen schon angegeben wurde, handelt es sich dabei im Gegensatz zu allen bisher bekannten Wundbehandlungssystemen, um ein System, welches ein Abführschlauch **4** umfasst, wobei Wundflüssigkeit **2** zurück zu der Wunde über einen Zuführschlauch **5** geführt wird.

Durch eine Vakuumpumpe **3** wird ein Unterdruck erzeugt, sodass die Wundflüssigkeit **2** in den Abführschlauch **4** eingesaugt wird. Die Wundflüssigkeit **2** umfasst u. a. Endotoxine und Bakterien, die für die Wundheilung schädlich sein können. Daher wird ein Endotoxinabsorber **10** eingebaut, der eine Matrix umfasst, dessen Oberfläche vorzugsweise Cholestyramin und/oder Anionenaustauschergruppierungen aus Polykationen und hydrophobe Eigenschaften umfasst, sodass Endotoxine besonders gut an die Oberfläche des Endotoxinabsorbers **10** gebunden werden. Damit werden die Endotoxine aus der Wundflüssigkeit **2** entfernt.

Bakterien, die durch den Endotoxinabsorber **10** diffundieren können, werden durch einen Bakterienfilter **6** gefiltert. Somit wird die Wundflüssigkeit **2** besonders gut gereinigt. Ein Silikonkissen **9** als Wundeinlage wird auf einen Wundgrund **12** gelegt, sodass die Wunde abgedeckt wird. Insbesondere hat ein Silikonkissen den Vorteil, dass es an die Wunde bzw. an die umgebende Haut **11** haftet. Damit verwächst sich nicht die zusammenschließende Haut während des Heilungsprozesses mit dem Silikonkissen **11.** Nachdem die Wundflüssigkeit **2** den Bakterienfilter passiert hat, gelangt es in den Zuführschlauch **5.** Ein Dreiwegeanschluss ist ebenso in der **Fig. 1** dargestellt, sodass ein Anteil der Wundflüssigkeiten **2** in ein Wundflüssigkeitsreservoir **7** und der restliche Anteil über den Zuführschlauch **5** in die Wunde gelangt. Dabei ist eine Wundabdeckungsfolie **8** ebenso umfasst, die auf die Wunde gelegt wird an die umgebende Haut **11** beklebt wird. Die Wundabdeckungsfolie **8** sorgt für einen luftdichten Verschluss der Wunde, sodass die Unterdruckwirkung der Vakuumpumpe **3** sich tatsächlich entfalten kann. Des Weiteren wird durch die Wundabdeckungsfolie **8** verhindert, dass zusätzliche Keime, Bakterien oder andere Fremdkörper in die Wunde gelangen.

Es hat sich gezeigt, dass das erfindungsgemäße Wundbehandlungssystem besonders vorteilhaft ist, da durch den Endotoxinfilter **10** Endotoxine aus der Wunde eliminiert werden können. Auch weitere Mikroorganismen wie Bakterien werden durch den Bakterienfilter **6** entfernt. Es ist auch von Vorteil, dass vorzugsweise ein Anteil der Wundflüssigkeit **2** zurück in die Wunde gelangt, da die Wundflüssigkeit **2** - wie eingangs erläutert - entscheidend ist für die Wundheilung. Ein weiterer Vorteil ist es, dass das Silikonkissen **9** als Wundeinlage benutzt wird, da es nicht an die Wunde haftet.

### BEZUGSZEICHENLISTE

- 1: Wundbehandlungssystem
- 2: Wundflüssigkeit
- 3: Absaugeinrichtung, Vakuumpumpe
- 4: Wundflüssigkeitszuführschlauch
- 6: Filter
- 7: Wundflüssigkeitsreservoir
- 8: Wundabdeckungsfolie
- 9: Wundeinlage
- 10: Endotoxinabsorber
- 11: Haut
- 12: Wundgrund

### LITERATURVERZEICHNIS

[1] Seidel, D. (2016) *Produkte zur Bakterientoxinbindung und Elimination bei der Behandlung von örtlichen Infektionen und ihre Herstellung* (EP 2 683 418 B1). Europäisches Patentamt. *https.-*//*t1p. de*/*muhz*
[2] Scheithauer, M. O., & Riechelmann, H. (2003). Grundlagen der kutanen Wundheilung. Übersicht Teil I. Laryngo-Rhino-Otol, 82, 31-35.
[3] Ding, Y., Sun, Z., Shi, R., Cui, H., Liu, Y., Mao, H., ... & Yan, F. (2018). Integrated endotoxin adsorption and antibacterial properties of cationic polyurethane foams for wound healing. ACS applied materials & interfaces, 11(3), 2860-2869.
[4] Seidel, D. (2004) *Vorrichtung zur Entfernung von bakteriellen Lipopolysacchariden oder*/*und Lipoteichonsäuren aus proteinhaltigen Flüssigkeiten sowie deren Verwendung zur Behandlung von Sepsis* (DE 102 58 944 A1)
[5] Razdan, S., Wang, J. C., & Barua, S. (2019). PolyBall: A new adsorbent for the efficient removal of endotoxin from biopharmaceuticals. Scientific reports, 9(1), 1-15.
[6] Ongkudon, C. M., Chew, J. H., Liu, B., & Danquah, M. K. (2012). Chromatographic removal of endotoxins: a bioprocess engineer's perspective. International Scholarly Research Notices, 2012.
[7] Batista, P. D. O. M. D., Lopes, A. M., Mazzola, P. G., Yagui, C. D. O. R., Penna, T. C. V., & Pessoa Júnior, A. (2007). Methods of endotoxin removal from biological preparations: a review*.*
[8] Petsch, D., Deckwer, W. D., Anspach, F. B., Legallais, C., & Vijayalakshmi, M. (1998). Endotoxin removal with poly (ethyleneimine)-immobilized adsorbers: Sepharose 4B versus flat sheet and hollow fibre membranes. Journal of Chromatography B: Biomedical Sciences and Applications, 707(1-2), 121-130.

## Patentansprüche

1. Wundbehandlungssystem (1) zur Entfernung bakterieller Toxine aus der Wundflüssigkeit (2), umfassend einen an eine Absaugeinrichtung (3) angeschlossenen Wundflüssigkeitsabführschlauch (4), **dadurch gekennzeichnet,**
a. **dass** der Abführschlauch (4) eine Matrix zur Absorption von bakteriellen Lippolysachariden (LPS) oder/und Lipoteichonsäuren (LTA) umfasst oder mit einer Matrix verbunden ist, wobei die Matrix so angeordnet ist, dass die abgesaugte Wundflüssigkeit (2) über die Matrix geleitet wird,
und
b. **dass** der Abführschlauch (4) fluidisch mit einem Wundflüssigkeitszuführschlauch (5) verbundenen ist.

2. Wundbehandlungssystem (1) nach Anspruch 1, wobei die Oberfläche der Matrix Cholestyramin und/oder Anionenaustauschergruppierungen aus Polykationen und hydrophobe Eigenschaften aufweist.

3. Wundbehandlungssystem (1) nach dem vorhergehenden Anspruch, wobei die Anionenaustauschergruppierung ausgestaltet sind als Polykationenketten aus synthetischen oder/und halbsynthetischen oder/und natürlichen Polykationenketten, welche in linearer oder verzweigter Form vorliegen.

4. Wundbehandlungssystem (1) nach einem oder mehreren der vorhergehenden Ansprüche, wobei die Anionenaustauschergruppierungen Di- oder Trialkylaminoalkyl-, Di- oder Trialkylaminoaryl-, Di- oder Triarylaminoalkyl-, Di- oder Triarylaminoaryl-, Di- oder Trialkylammoniumalkyl-, Di- oder Triarylammoniumalkyl-, Di- oder Triarylammoniumaryl-, oder Di- oder Trialkyl- ammoniumaryl-Reste enthalten oder Polymere aus positiv geladenen oder tertiäre oder quartäre Aminogruppen enthaltenden Aminosäuren, wie Polylysin, Polyarginin oder Polyhistidin oder Mischpolymere oder Derivate hiervon oder Polyethylenimin sind.

5. Wundbehandlungssystem (1) nach einem oder mehreren der vorhergehenden Ansprüche, wobei die Anionenaustauschergruppierungen Trimethylbenzylammonium-Reste enthalten.

6. Wundbehandlungssystem (1) nach einem oder mehreren der vorhergehenden Ansprüche, wobei die Absaugeinrichtung eine Vakuumpumpe (3) ist.

7. Wundbehandlungssystem (1) nach einem oder mehreren der vorhergehenden Ansprüche, wobei der Abführschlauch (4) einen Filter (6) umfasst, wobei der Filter (6) so angeordnet ist, dass abgesaugte Wundflüssigkeit (2) vom Abführschlauch (4) durch den Filter (6) geleitet wird, und der Filter (6) bevorzugt zum Filtern von Bakterien geeignet ist.

8. Wundbehandlungssystem (1) nach einem oder mehreren der vorhergehenden Ansprüche, umfassend ein fluidisch mit dem Abführ- und Zuführschlauch verbundenes Wundflüssigkeitsreservoir (7).

9. Wundbehandlungssystem (1) nach dem vorhergehenden Anspruch, wobei das Wundflüssigkeitsreservoir (7), der Abführ- und der Zuführschlauch über einen Dreiwegeanschluss miteinander verbunden sind, wobei der Dreiwegeanschluss bevorzugt ein Ventil zur Leitung der Wundflüssigkeit (2) umfasst.

10. Wundbehandlungssystem (1) nach einem oder mehreren der vorhergehenden Ansprüche, umfassend eine Wundabdeckungsvorrichtung, die geeignet ist zur Aufnahme der Ab- und Zuführschläuche, wie beispielsweise eine Wundabdeckungsfolie (8).

11. Wundbehandlungssystem (1) nach einem oder mehreren der vorhergehenden Ansprüche umfassend eine Wundeinlage (9), wie beispielsweise einen Schwamm oder ein Silikonkissen.

12. Wundbehandlungssystem (1) nach einem oder mehreren der vorhergehenden Ansprüche, **dadurch gekennzeichnet, dass** der Abführschlauch (4) und der Zuführschlauch (5) so konfiguriert sind, dass die an Wunden innerhalb eines Körpers angebracht werden können, um Wundflüssigkeit (2) aus einer inneren Wunde durch den Abführschlauch (4) über die Matrix zu leiten, und anschließend durch den Zuführschlauch (5) an die innere Wunde zu befördern.

13. Kit zur Entfernung bakterieller Toxine aus einer Wundflüssigkeit (2) mit einem Wundbehandlungssystem (1) nach einem oder mehreren der vorhergehenden Ansprüche, umfassend:
a. ein Wundflüssigkeitsabführschlauch (4), wobei der Abführschlauch (4) eine Matrix zur Absorption von bakteriellen Lippolysachariden (LPS) oder/und Lipoteichonsäuren (LTA) umfasst, oder mit einer Matrix verbunden ist, wobei die Matrix so angeordnet ist, dass nach Zusammenbau des Wundbehandlungssystems die abgesaugte Wundflüssigkeit (2) über die Matrix geleitet wird,
b. ein Wundflüssigkeitszuführschlauch (5),
c. eine Wundabdeckungsvorrichtung, die geeignet ist zur Aufnahme der Ab- und Zuführschläuche, wie beispielsweise eine Wundabdeckungsfolie (8), und
d. optional ein Bakterienfilter (6), und
e. optional eine Wundeinlage (9), wie beispielsweise einen Schwamm oder ein Silikonkissen

14. Verfahren zur Entfernung bakterieller Toxine aus einer Wundflüssigkeit (2), umfassend die Verwendung eines Wundbehandlungssystems (1) und/oder eines Kits nach einem oder mehreren der vorhergehenden Ansprüche.
